(19) Europäisches Patentamt
European Patent Office
Office européen des brevets

(11) Veröffentlichungsnummer: **0 003 298**
**B1**

(12) **EUROPÄISCHE PATENTSCHRIFT**

(21) Anmeldenummer: **79100097.9**

(22) Anmeldetag: **15.01.79**

(51) Int. Cl.³: **C 07 D 235/28,**
C 07 D 235/02,
A 61 K 31/415

(54) **4-Hydroxy-2-benzimidazolin-thion-Derivate, Verfahren zu deren Herstellung sowie diese Verbindungen enthaltende Arzneimittel.**

(30) Priorität: **18.01.78 DE 2801980**

(43) Veröffentlichungstag der Anmeldung:
**08.08.79 Patentblatt 79/16**

(45) Bekanntmachung des Hinweises auf die Patenterteilung:
**25.03.81 Patentblatt 81/12**

(84) Benannte Vertragsstaaten:
**BE CH DE FR GB IT LU NL SE**

(56) Entgegenhaltungen:
**DE - A - 2 700 193**
**DE - A - 2 819 458**
**FR - A - 1 583 153**

(73) Patentinhaber: **BOEHRINGER MANNHEIM GMBH**
**Sandhofer Strasse 112-132 Postfach 31 01 20**
**D-6800 Mannheim 31-Waldhof (DE)**

(72) Erfinder: **Ross, Carl-Heinz, Dr.**
**Rathausstrasse 45**
**D-6806 Viernheim (DE)**

(72) Erfinder: **Friebe, Walter-Gunar, Dr.**
**Oberstrasse 13**
**D-6100 Darmstadt (DE)**

(72) Erfinder: **Kampe, Wolfgang, Dr.**
**Zedernstrasse 49**
**D-6805 Heddesheim (DE)**

(72) Erfinder: **Bartsch, Wolfgang, Dr.**
**Franconviller-Strasse 5**
**D-6806 Viernheim (DE)**

(72) Erfinder: **Roesch, Egon, Dr.**
**Am oberen Luisenpark 22**
**D-6800 Mannheim 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 003 298 B1

4-Hydroxy-2-benzimidazolin-thion-Derivate, Verfahren zu deren Herstellung sowie diese Verbindungen enthaltende Arzneimittel

Die vorliegende Erfindung betrifft neue 4-Hydroxy-2-benzimidazolin-thion-Derivate, Verfahren zu deren Herstellung sowie diese Verbindungen enthaltende Arzneimittel.

Die neuen Verbindungen sowie ihre pharmakologisch unbedenklichen Salze bewirken eine Hemmung adrenergischer $\beta$-Rezeptoren und eignen sich daher zur Behandlung oder Prophylaxe bei Herz- und Kreislauferkrankungen.

In der DE—OS 2700193 sind 4-Hydroxy-2-benzimidazolinon-Derivate mit $\beta$-Rezeptorenblockierender Wirkung beschrieben.

Die vorliegende Erfindung betrifft basisch substituierte Derivate des 4-Hydroxy-2-benzimidazolinthions der allgemeinen Formel I

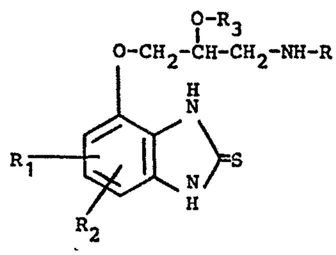

(I)

in der

R einen niederen Alkylrest,

$R_1$ und $R_2$, die gleich oder verschieden sind, jeweils ein Wasserstoffatom oder einen niederen, geradkettigen oder verzweigten Alkylrest oder $R_1$ und $R_2$ gemeinsam einen Alkylenrest und

$R_3$ ein Wasserstoffatom oder einen Acylrest.

bedeuten, deren pharmakologisch verträgliche Salze, Verfahren zur Herstellung derselben sowie pharmazeutische Zubereitungen, die diese Substanzen enthalten.

Die Verbindungen der allgemeinen Formel I enthalten in der Aminopropoxy-Seitenkette ein optisch aktives Kohlenstoffatom und können daher sowohl in einer racemischen als auch in zwei optisch aktiven Formen auftreten. Gegenstand der vorliegenden Anmeldung sind sowohl die racemischen Formen als auch die optischen Isomeren.

Die niederen Alkylgruppen, die in den Definitionen der Substituenten R, $R_1$ und $R_2$ auftreten, können 1 bis 6, vorzugsweise 1 bis 4 Kohlenstoffatom enthalten. Bevorzugt sind die Methyl-, Isopropyl- und tert-Butylgruppe.

Der Alkylenrest, der gegebenenfalls durch die Substituenten $R_1$ und $R_2$ gebildet werden kann, enthält 2 bis 4 Kohlenstoffatome.

Die Acylgruppen $R_3$ können Säurereste geradkettiger oder verzweigter aliphatischer Carbonsäuren mit 2 bis 6 Kohlenstoffatomen oder aromatischer, gegebenenfalls durch Halogenatome, niedere Alkyl- oder niedere Alkoxygruppen substituierter Carbonsäuren sein. Bevorzugt sind der Acetyl-, Pivaloyl- und Benzoylrest.

Die neuen Verbindungen sowie ihre pharmakologisch unbedenklichen Salze weisen im Vergleich zum Stand der Technik und zu Handelsprodukten mit ähnlicher Konstitution und Wirkung eine bessere $\beta$-Rezeptoren-blockierende Wirkung auf.

Zum Beweise hierfür wurde im pharmakologischen Test als Vertreter der erfindungsgemäßen Substanzen die Verbindung des Beispiels 1, das 4 - (2 - Hydroxy - 3 - tert - butylamino - propoxy) - 2 - benzimidazolin - thion mit dem 4 - (2 - Hydroxy - 3 - tert - butylaminopropoxy) - benzimidazol - 2 - on aus der DE—OS 2700193 und dem Handelsprodukt Propranolol = 1-Isopropylamino-3-(1-naphthyl)-2-propanol verglichen.

Als Maß für die $\beta$-Rezeptoren-Blockade wurde an wachen Kaninchen die $DE_{250}$ bestimmt, d. h. die Dosis des $\beta$-Blockers, bei der die Herzfrequenz nach Injektion von Isoprenalin nur noch auf 250 Schläge/min anstieg.

Die Auswertung der Versuche ergab, daß zur Steigerung der Isoprenalin-induzierten Herzfrequenz auf 250 Schläge/min unter gleichen Versuchsbedingungen von dem Handelspräparat die über 200-fache Dosis und von der aus der DE—OS 2700193 bekannten Vergleichsverbindung etwa die vierfache Dosis im Vergleich mit der ausgewählten erfindungsgemäßen Verbindung erforderlich ist.

Andere erfindungsgemäße Verbindungen zeigen gleiche, zum Teil noch bessere Versuchsergebnisse.

Die Herstellung der neuen Verbindungen der allgemeinen Formel I ist dadurch gekennzeichnet, daß man in an sich bekannter Weise entweder.

a) eine Verbindung der allgemeinen Formel II

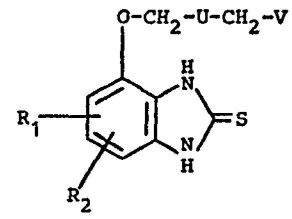

(II)

mit einer Verbindung der allgemeinen Formel III

W—R (III)

umsetzt, in denen R, $R_1$ und $R_2$ die oben angegebene Bedeutung haben, einer der Rest V und W eine Aminogruppe und der andere einen reaktiven Rest darstellen, U für di Gruppe

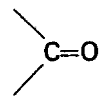

oder

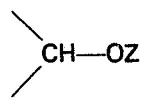

steht, wobei Z die Bedeutung des Substituenten $R_3$ besitzt oder auch zusammen mit V eine Einfachbindung sein kann, wobei W dann eine Aminogruppe bedeutet.
und
falls U die Gruppe

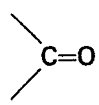

bedeutet, anschließend reduziert oder

b) eine Verbindung der allgemeinen Formel IV

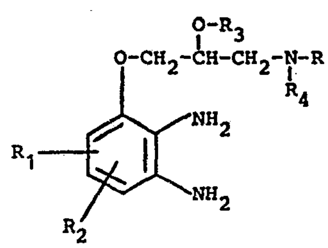

(IV)

mit einer Verbindung der allgemeinen Formel V

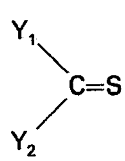

(V)

umsetzt, in denen R, $R_1$, $R_2$ und $R_3$ die oben angegebene Bedeutung haben, während $R_4$ ein Wasserstoffatom oder eine abspaltbare Schutzgruppe darstellt, $Y_1$ und $Y_2$ gleich oder verschieden sind und jeweils einen reaktiven Rest beschreiben,

und anschließend eine gegebenenfalls, vorhandene Schutzgruppe $R_4$ abspaltet,
oder

c) eine Verbindung der allgemeinen Formel VI

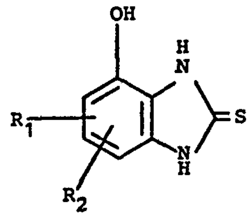

(VI)

mit einer Verbindung der allgemeinen Formel VII

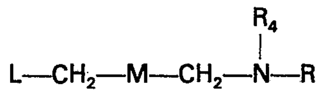

(VII)

umsetzt,

in denen R, $R_1$, $R_2$ und $R_4$ die oben angegebene Bedeutung haben, während M für die Gruppen

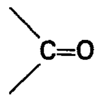

oder

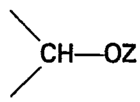

steht, wobei Z die Bedeutung des Substituenten $R_3$ besitzt oder auch zusammen mit L eine Einfachbindung sein kann und der Rest L einen reaktiven Rest darstellt,

falls M die Gruppe

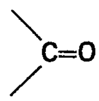

bedeutet, anschließend reduziert und eine gegebenenfalls vorhandene Schutzgruppe $R_4$ abspaltet

und anschließend an die Umsetzungen gegebenenfalls Verbindungen der allgemeinen Formel I in pharmakologisch verträgliche Salze überführt, wobei man gegebenenfalls vorher in Verbindungen der allgemeinen Formel I, in denen $R_3$ Wasserstoff bedeutet, die Hydroxygruppe acyliert.

$Y_1$ und $Y_2$ in Verbindungen der allgemeinen Formel V stehen für alle Reste, die mit den beiden primären Aminogruppen in Verbindungen der allgemeinen Formel IV zum Imidazolinring reagieren können. Solche Reste sind vorzugsweise Halogenatome, wie Brom oder Chlor, Amino-, Imidazolyl-, Niederalkoxy-, niedere Acyloxy-, Mercapto- und Niederalkoxythiocarbonylgruppen. Beispielsweise können als Verbindungen der allgemeinen Formel V Thiocarbonylhalogenide, Thioharnstoff oder auch Xanthogenate eingesetzt werden. Verbindungen der allgemeinen Formel V können auch in situ aus anderen Verbindungen, z. B. Schwefelkohlenstoff in alkalischer Lösung, in der Reaktionsmischung hergestellt werden.

Die erfindungsgemäßen Verfahren werden zweckmäßig in einem unter den Reaktionsbedingungen inerten Lösungsmittel, z. B. Wasser, Ethanol, Dioxan oder Dimethylformamide, gegebenenfalls in Gegenwart eines säurebindenden Mittels, durchgeführt. Die Umsetzungen können auch nach Mischen der Reaktionskomponenten ohne Lösungsmittel erreicht werden. Die Reaktionen werden durch Stehenlassen bei Raumtemperatur oder durch Erwärmen, gegebenenfalls unter einer Schutzgasathmosphäre, ausgeführt.

Die gegebenenfalls durchzuführende Reduktion der Gruppe

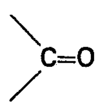

erfolgt durch katalytische Hydrierung mit Edelmetall- oder Nickelkatalysatoren oder mittels komplexer Metallhydride wie z. B. Natriumborhydrid.

Als leicht abspaltbare Schutzgruppen können im Prinzip alle in der Peptidchemie zum intermediären Schutz von Aminogruppen verwendeten Schutzgruppen eingesetzt werden, die nach erfolgter Umsetzung entfernt werden können. Mit großem Vorteil werden bei der vorliegenden Umsetzung gemäß Verfahren b) und c) die Benzyl- und Carbobenzoxygruppe eingeführt, die sich nach der Reaktion der Verbindungen der allgemeinen Formel IV bzw. VI mit Substanzen der allgemeinen Formel V bzw. VII ohne weiteres in an sich bekannter Weise hydrogenolytisch abspalten lassen.

Die Verbindungen der allgemeinen Formel IV können beispielsweise hergestellt werden, indem man Verbindungen der allgemeinen formel VIII

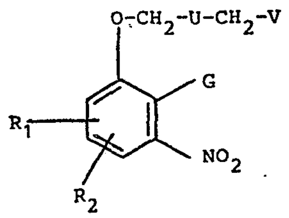

(VIII)

in der $R_1$, $R_2$, U und V die oben angegebene Bedeutung haben, während G für die Amino- oder Nitrogruppe steht,

mit Verbindungen der allgemeinen Formel III analog Verfahren a) umsetzt und die so erhaltenen Substanzen reduziert. Die Reduktion erfolgt in an sich bekannter Weise, vorzugsweise durch katalytische Hydrierung. Die hierbei entstehenden rohen o-Phenylendiaminderivate der allgemeinen Formel IV werden vorteilhaft ohne weitere Reinigung als mineralsaure Salze als Ausgangsprodukte beim Verfahren b) eingesetzt.

Die Verbindungen der allgemeinen Formel VIII sind entweder beschriebene Substanzen oder können leicht aus beschriebenen Substanzen nach bekannten Methoden hergestellt werden.

Die gegebenenfalls durchzuführende nachträgliche Acylierung von Verbindungen der allgemeinen Formel I, in der $R_3$ ein Wasserstoffatom bedeutet, kann in üblicher Weise durch Umsetzung mit einem reaktiven Säurederivat, z. B. Säurehalogenid, Säureazid oder Säureanhydrid, gegebenenfalls in Gegenwart eines säurebindenden Mittels, z. B. Pyridin, in einem Lösungsmittel, z. B. Aceton, Benzol, Dimethylformamid, oder auch in überschüssiger Säure erfolgen.

Die erfindungsgemäßen Verbindungen der Formel I können in Form eines racemischen Gemisches anfallen. Die Trennung des Racemats in die optisch aktiven Formen geschieht nach an sich bekannten Methoden über die diastereomeren Salze aktiver Säuren; wie z. B. Weinsäure, Äpfelsäure oder Camphersulfonsäure.

Die neuen Verbindungen der allgemeinen Formel I fallen unter den Reaktionsbedingungen der beschriebenen Verfahren vorwiegend als Säureadditionssalze an, z. B. als Hydrochloride, und können nach beschriebenen Methoden ohne weiteres in die freien Basen überführt werden.

Zur Überführung der Verbindungen der allgemeinen Formel I in ihre pharmakologisch unbedenklichen Salze setzt man diese, vorzugsweise in einem organischen Lösungsmittel, mit der äquivalenten Menge einer anorganischen oder organischen Säure, z. B. Salzsäure, Bromwasserstoffsäure, Phosphorsäure, Schwefelsäure, Essigsäure, Citronensäure, Maleinsäure, um.

Zur Herstellung von Arzneimitteln werden die Substanzen I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z. B. Olivenöl, suspendiert oder gelöst.

Die erfindungsgemäßen neuen Substanzen I und ihre Salze können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungsmittel, Lösungsvermittler oder Puffer enthält. Derartige Zusätze sind z.B. Tartrat- und Citratpuffer, Ethanol, Komplexbildner (wie Ethylendiamintetraessigsäure und deren nicht-toxischen Salze), hochmolekulare Polymere (wie flüssiges Polyethylenoxyd) zur Viskositätsregulierung. Feste Trägerstoffe sind z. B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette und feste hochmolekulare Polymere (wie Polyethylenglykole). Für orale Applikation geeignete Zubereitung können gewünschtenfalls Geschmacks- und Süßstoffe enthalten.

Bevorzugt im Sinne der vorliegenden Anmeldung sind außer den in den Beispielen genannten Verbindungen die folgenden:

4-(2-Benzoyloxy-3-tert-butylamino-propoxy)-6-methyl-2-benzimidazolinthion
4-(2-Hydroxy-3-tert-butylamino-propoxy)-6,7-cyclopentenobenzimidazolinthion
7-tert-Butyl-4-(2-hydroxy-3-tert-butylamino-propoxy)-2-benzimidazolinthion.

Die Erfindung wird durch die folgenden Beispiele näher erläutert. Sie zeigen einige der zahlreichen möglichen Verfahrensvarianten, die zur Synthese der erfindungsgemäßen Verbindungen verwendet werden können. Sie stellen jedoch keine Einschränkung des Erfindungsgegenstandes dar.

Beispiel 1

*4-(2-Hydroxy-3-tert-butylamino-propoxy)-2-benzimidazolin-thion*

7,2 g 2,3-Diamino-1-(2-hydroxy-3-tert-butylamino-propoxy)-benzol (Lit.: DE—OS 2432269) werden in 20 ml Ethanol gelöst und mit 1,26 g Kaliumhydroxid, 1,73 g Schwefelkohlenstoff und 2,9 ml Wasser versetzt. Man kocht 3 Stunden unter Rückfluß. Nach Klärung mit Aktivkohle wird die 70°C heiße Lösung mit 1,6 ml Eisessig in 23 ml Wasser versetzt und zur Trockene eingeengt. Aus Alkohol kristallisieren 2,4 g (40%) freie Base vom Fp. 214—216°C, die in ethanolischer Lösung mit etherischer Salzsäure in das Hydrochlorid vom Fp. 288—289°C überführt wird.

Beispiel 2

*4-[2-Hydroxy-3-(2-propylamino)-propoxy]-2-benzimidazolin-thion*

Die Titelverbindung als Hydrochlorid vom Fp. 254°C erhält man dem in Beispiel 1 beschriebenen Verfahren folgend aus 2,3-Diamino-1-[2-hydroxy-3-(2-propylamino)-propoxy]-benzol.

Beispiel 3

*4-(2-Hydroxy-3-tert-butylamino-propoxy)-6-methyl-2-benzimidazolin-thion*

14,3 g 2,3-Diamino-1-(2-hydroxy-3-tert-butylamino-propoxy)-5-methyl-benzol (erhalten aus dem Trihydrochlorid durch Versetzen mit der stoechiometrischen Menge Natriummethylatlösung und Einengen), 9,4 g Kaliumxanthogenat, 55 ml Ethanol und 8 ml Wasser werden 10 Stunden unter Rückfluß gekocht. Nach Versetzen mit 12 ml konz. Salzsäure kocht man nochmals kurz auf und engt zur Trockene ein. Nach Umkristallisieren aus 500 ml Wasser und 100 ml Dimethylformamid unter Zusatz von Aktivkohle erhält man 10,2 g (55% d. Th.) farblose Kristalle vom Fp. 295—296°C (unter Zersetzung) als Hydrochlorid.

Die als Zwischenprodukt benötigte Diaminoverbindung kann auf folgendem Weg dargestellt werden:

Durch Nitrierung von vorher acetyliertem 2-Amino-5-methyl-phenol (Fp. 156—158°C) bei 30°C mit Acetanhydrid in Eisessig erhält man 1-Acetoxy-2-acetamido-3-nitro-5-methyl-benzol mit Fp. 163—165°C.

Verseifen mit 2 N Salzsäure liefert 2-Amino-5-methyl-3-nitrophenol vom Fp. 197—199°C.

Aus der vorstehenden Verbindung erhält man nach Umsetzen mit 3-chlor-1,2-epoxypropan und 25proz. Natronlauge bei 75°C das 2-(2,3-Epoxy-propoxy)-4-methyl-6-nitro-anilin mit Fp. 90—91°C.

Reaktion der vorstehenden Verbindung mit tert.-Butylamin in Ethanol und anschließende katalytische Hydrierung über Platindioxid führt nach Ansäuern mit Salzsäure zu amorphem 2,3-diamino-1-(2-hydroxy-3-tert-butylamino-propoxy)-5-methyl-benzoltrihydrochlorid.

Beispiel 4

*4-[2-Hydroxy-3-(2-propylamino)-propoxy]-6-methyl-2-benzimidazolin-thion*

Aus Kaliumxanthogenat und 2,3-Diamino-1-[2-hydroxy-3-(2-propylamino)-propoxy]-5-methylbenzol wird gemäß dem in Beispiel 3 ausgeführten Verfahren die Titelverbindung vom Zersetzungspunkt 294—295°C (aus Ethanol) hergestellt.

Das als Zwischenprodukt eingesetzte Diamin wird durch Umsetzung des in Beispiel 3 beschriebenen 2-(2,3-Epoxy-propoxy)-4-methyl-6-nitroanilins mit 2-Propylamin zu 2-[2-Hydroxy-3-(2-propylamino)-propoxy]-4-methyl-6-nitroanilin und anschließende katalytische Hydrierung erhalten.

Beispiel 5

*4-(2-Pivaloyloxy-3-tert-butylamino-propoxy)-6-methyl-2-benzimidazolin-thion*

3,45 g 4-(2-Hydroxy-3-tert-butylamino-propoxy)-6-methyl-2-benzimidazolin-thion-hydrochlorid (Beispiel 3) überführt man mit der stoechiometrischen Menge methanolischen Natriummethylats in die freie Base. Diese wird mit 12 g Pivalinsäureanhydrid in 80 ml Dimethylformamid 4 Tage bei Raumtemperatur umgesetzt. Die kristalline Abscheidung wird mit etherischer Salzsäure behandelt und nach Einengen aus Ethanol umkristallisiert. Die Titelverbindung schmilzt bei 294—296°C (als Hydrochlorid).

Beispiel 6

*4-(2-Hydroxy-3-tert-butylamino-propoxy)-7-methyl-2-benzimidazolin-thion*

Analog dem in Beispiel 3 angegebenen Verfahren erhält man aus dem 2,3-Diamino-1-(2-hydroxy-3-tert-butylamino-propoxy)-4-methyl-benzol die Titelverbindung vom Zersetzungspunkt 298—299°C (aus Ethanol) als Hydrochlorid.

Die im obiger Reaktion eingesetzte Zwischenstufe kann auf folgendem Weg hergestellt werden:

2,3-Dinitro-4-methyl-phenol (H. E. Dadswell und J. Kenner, J. Chem Soc. *1927,* 583) wird mit 3-Chlor-1,2-epoxypropan und 25proz. Natronlauge umgesetzt. Man erhält in 78% Ausbeute 2,3-Dinitro-1-(2,3-epoxy-propoxy)-4-methyl-benzol vom Fp. 121—123°C.

Die vorstehende Verbindung wird in siedendem Ethanol mit tert-Butylamin zu 2,3-Dinitro-1-(2-hydroxy-3-tert-butylaminopropoxy)-4-methyl-benzol mit Fp. 104—106°C umgesetzt.

Durch katalytische Hydrierung über Platindioxid erhält man daraus amorphes 2,3-Diamino-1-(2-hydroxy-3-tert-butylaminopropoxy)-4-methyl-benzol, das als Trihydrochlorid isoliert wird.

Beispiel 7

*6-tert-Butyl-4-(2-hydroxy-3-tert-butylamino-propoxy)-2-benzimidazolin-thion*

Aus 5-tert-Butyl-2,3-diamino-1-(2-hydroxy-3-tert-butylaminopropoxy)-benzol wird analog dem in Beispiel 3 angegebenen Verfahren die Titelverbindung vom Zersetzungspunkt oberhalb 280°C erhalten (als Hydrochlorid).

Die als Zwischenprodukt verwendete Diaminoverbindung kann über folgende Stufen synthetisiert werden:

Durch Reaktion von 4-tert-Butyl-2-(2,-epoxy-propoxy)-6-nitroanilin mit tert-Butylamin erhält man 4-tert-Butyl-2-(2-hydroxy-3-tert-butylamino-propoxy)-6-nitro-anilin, das als Hydrochlorid vom Zersetzungspunkt 115—120°C isoliert wird.

Die Hydrierung der vorstehenden Verbindung in ethanolischer Lösung über Platindioxid ergibt 5-tert-Butyl-2,3-diamino-1-(2-hydroxy-3-tert-butylamino-propoxy)-benzol, das als amorphes Trihydrochlorid isoliert wird.

Beispiel 8

*6,7-Dimethyl-4-(2-hydroxy-3-tert-butylamino-propoxy)-2-benzimidazolin-thion*

Analog dem in Beispiel 3 angegebenen Verfahren erhält man aus 2,3-Diamino-4,5-dimethyl-1-(2-hydroxy-3-tert-butylaminopropoxy)-benzol and Kaliumxanthogenat die Titelverbindung vom Zersetzungspunkt 307—308°C (als Hydrochlorid).

Die als Zwischenstufe benötigte Diaminoverbindung wird auf folgendem Wege aus dem 2-Amino-4,5-dimethyl-phenol [Lit.: E. Diepolder, Chem. Ber. *42,* 2916 (1909)] synthetisiert:

Man acetyliert das vorstehende Phenol mit Acetanhydrid in Essigester/Pyridin zu 2-Acetamido-1-acetoxy-4,5-dimethylbenzol (Fp. 156—158°C), das in Acetanhydrid mit 100proz. Salpetersäure in Acetanhydrid bei 20°C nitriert wird. Aus dem Nitriergemisch isoliert man in 43% Ausbeute das 2-Acetamido-1-acetoxy-4,5-dimethyl-3-nitro-benzol vom Fp. 209—211°C.

Nach Verseifen oder vorstehenden Verbindung in 2 N Salzsäure isoliert man 2-Amino-4,5-dimethyi-3-nitro-phenol mit Fp. 176—178°C.

Die vorstehende Verbindung wird mit methanolischem Natriummethylat in das Natrium-Salz überführt. Dieses wird in Dioxan/Dimethylformamid mit überschüssigem 3-Chlor-1,2-epoxypropan bei 75°C umgesetzt. Nach Einengen wird der Rückstand in Chloroform aufgenommen, mit Wasser und Aktivkohle behandelt und vom Lösungsmittel befreit.

Dieser amorphe Rückstand von 2-Amino-4,5-dimethyl-1-(2,3-epoxy-propoxy)-3-nitro-benzol reagiert in siedendem Ethanol mit tert-Butylamin zu 2-Amino-4,5-dimethyl-1-(2-hydroxy-3-tert-butylamino-propoxy)-3-nitro-benzol.

Die vorstehende Verbindung wird quantitativ in Ethanol über Platindioxid zu 2,3-Diamino-4,5-dimethyl-1-(2-hydroxy-3-tert-butylamino-propoxy)-benzol hydriert, das als Trihydrochlorid isoliert wird.

**Patentansprüche**

1. 4-Hydroxy-2-benzimidazolin-thion-Derivate der allgemeinen Formel I

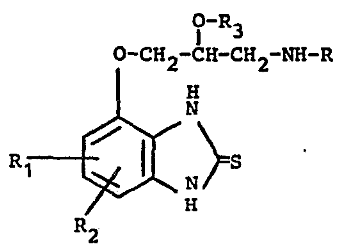

(I)

in der

R einen niederen Alkylrest,

$R_1$ und $R_2$, die gleich oder verschieden sind, jeweils ein Wasserstoffatom oder einen niederen, geradkettigen oder verzweigten Alkylrest oder

$R_1$ und $R_2$ gemeinsam einen Alkylenrest mit 2 bis 4 kohlenstoffatomen

und

$R_3$ Wasserstoff oder einen Acylrest

bedeuten,

sowie deren pharmakologisch unbedenkliche Salze.

2. Verfahren zur Herstellung von 4-Hydroxy-2-benzimidazolinthion-Derivaten gemäß Anspruch 1 mit den dort angegebenen Definitionen sowie deren pharmakologisch unbedenklichen Salzen, dadurch gekennzeichnet, daß man in an sich bekannter Weise entweder.

a) eine Verbindung der allgemeinen Formel II

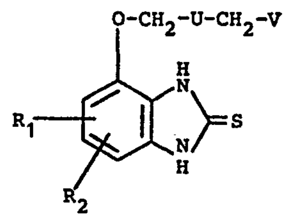

(II)

mit einer Verbindung der allgemeinen Formel III

W—R (III)

umsetzt,

in denen einer der Reste V und W eine Aminogruppe und der andere einen reaktiven Rest darstellen, U für die Gruppe

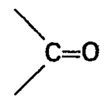

oder

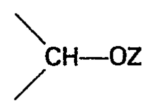

steht, wobei Z die Bedeutung des Substituenten $R_3$ besitzt oder auch zusammen mit V eine Einfachbindung sein kann, wobei W dann eine Aminogruppe bedeutet

und

falls U die Gruppe

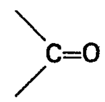

bedeutet, anschließend reduziert

oder

b) eine Verbindung der allgemeinen Formel IV

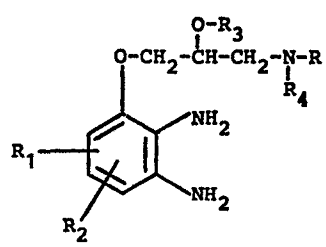

(IV)

mit einer Verbindung der allgemeinen Formel V

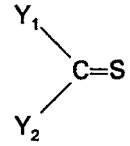

(V)

umsetzt,

in denen $R_4$ ein Wasserstoffatom oder eine abspaltbare Schutzgruppe darstellt, $Y_1$ and $Y_2$ gleich oder verschieden sind und jeweils einen reaktiven Rest beschreiben,

und anschließend eine gegebenenfalls vorhandene Schutzgruppe $R_4$ abspaltet,

oder

c) eine Verbindung der allgemeinen Formel VI

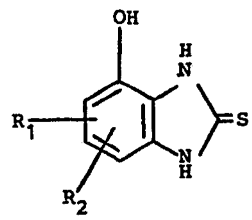

(VI)

mit einer Verbindung der allgemeinen Formel VII

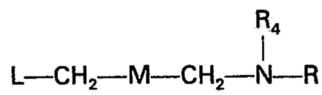

(VII)

umsetzt,

in denen M für die Gruppen

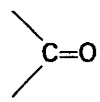

oder

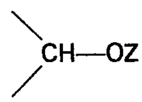

steht, wobei Z die Bedeutung des Substituenten $R_3$ besitzt oder auch zusammen mit L eine Einfachbindung sein kann und der Rest L einen reaktiven Rest darstellt,

falls M die Gruppe

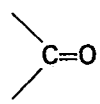

bedeutet, anschließend reduziert und eine gegebenenfalls vorhandene Schutzgruppe $R_4$ abspaltet und anschließend an die Umsetzungen gegebenenfalls Verbindungen der allgemeinen Formel I in pharmakologisch verträgliche Salze überführt, wobei man gegebenenfalls vorher in Verbindungen der allgemeinen Formel I, in denen $R_3$ Wasserstoff bedeutet, die Hydroxygruppe acyliert.

3. Arzneimittel enthaltend eine Verbindung gemäß Anspruch 1 sowie an sich bekannte pharmakologisch unbedenkliche Trägerund Hilfsstoffe.

4. Verbindungen gemäß Anspruch 1 oder deren Salze zur Verwendung in der Behandlung oder Prophylaxe bei Herz- und Kreislauferkrankungen.

**Claims**

1. 4-Hydroxy-2-benzimidazoline-thione derivatives of the general formula I:

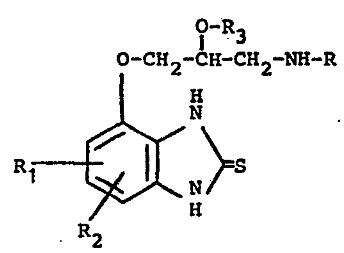

(I)

wherein R represents a lower alkyl radical, $R_1$ and $R_2$, which are the same or different, each represent a hydrogen atom or a lower straight-chained or branched alkyl radical or $R_1$ and $R_2$ together represent an alkylene radical with 2 to 4 carbon atoms and $R_3$ represents hydrogen or an acyl radical; as well as their pharmacologically acceptable salts.

2. Process for the preparation of 4-hydroxy-2-benzimidazoline-thione derivatives according to claim 1 with the definitions given therein, as well as of their pharmacologically acceptable salts,

characterised in that, in per se known manner, one either

a) reacts a compound of the general formula II:

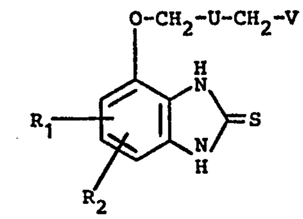

(II)

with a compound of the general formula III:

W—R (III)

in which one of the residues V and M represents an amino group and the other a reactive residue, U stands for the group

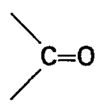

or

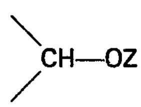

whereby Z possesses the meaning of the substituent $R_3$ or, together with V, can also be a single bond, whereby W then represents an amino group, and, if U represents the

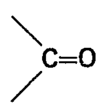

group, one subsequently reduces or

b) reacts a compound of the general formula IV:

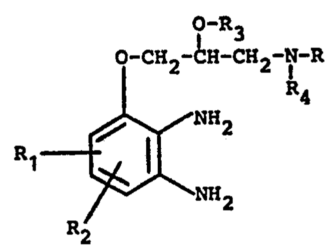

(IV)

with a compound of the general formula V:

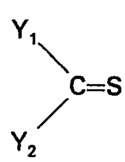

(V)

in which $R_4$ represents a hydrogen atom or a protective group which can be split off, $Y_1$ and $Y_2$ are the same or different and each represents a reactive residue, and subsequently splits off a protective group $R_4$ possibly present, or

c) reacts a compound of the general formula VI:

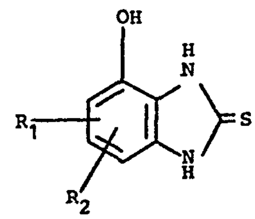

(VI)

with a compound of the general formula VII:

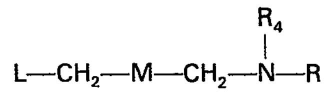

(VII)

in which M stands for the groups

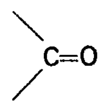

or

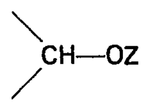

whereby Z possesses the meaning of the substituent $R_3$ or, together with L, can also represent a single bond, and the residue L represents a reactive residue, when M represents the

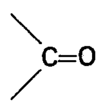

group, subsequently reduces and a protective group $R_4$ possibly present is again split off; and subsequent to the reaction possibly converts compounds of the general formula I into pharmacologically acceptable salts, whereby, in compounds of the general formula I, in which $R_3$ signifies hydrogen, one possibly previously acylates the hydroxyl group.

3. Medicaments comprising a compound according to claim 1, as well as per se known pharmacologically acceptable carrier and adjuvant materials.

4. Compounds according to claim 1 or their salts for use in the treatment or prophylaxis of heart and circulatory diseases.

**Revendications**

1. Dérivés de la 4-hydroxy-2-benzimidazoline-thione de formule générale I

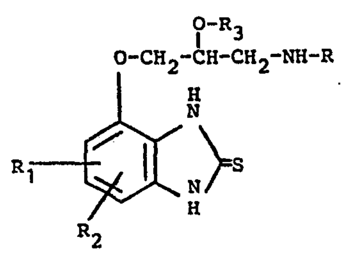

(I)

dans laquelle:

R est un reste alkyle inférieur,

$R_1$ et $R_2$, qui sont identiques ou différents, sont chacun un atome d'hydrogène ou un reste alkyle inférieur, à chaîne droite ou ramifiée, ou bien,

$R_1$ et $R_2$ forment ensemble un reste alkylène ayant de 2 à 4 atomes de carbone, et,

$R_3$ est de l'hydrogène ou un reste acyle,

ainsi que leurs sels pharmacologiquement inoffensifs.

2. Procédé pour la préparation de dérivés de la 4-hydroxy-2-benzimidazoline-thione définis dans la revendication 1, ainsi que leurs sels pharmacologiquement inoffensifs, caractérisé en ce que l'on fait réagir de façon en soi connue soit:

a) un composé de formule générale II

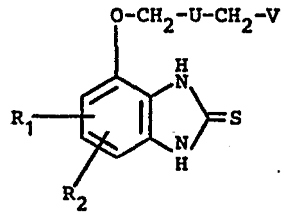

(II)

avec un composé de formule générale III

W—R (III)

dans lesquelles l'un des restes V et W est un groupe aminogène et l'autre un reste réactif, U est le groupe

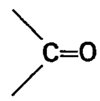

ou

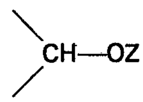

où Z a la même signification que le substituant $R_3$ ou bien forme ensemble avec V une simple liaison, auquel ca W est un groupe aminogène, et,

dans le où U est le groupe

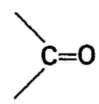

on soumet le produit réactionnel à une réduction, soit

b) un composé de formule générale IV

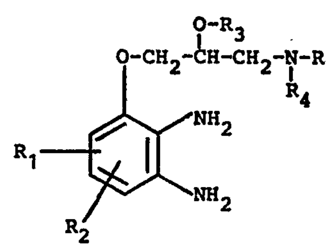

(IV)

avec un composé de formule générale V

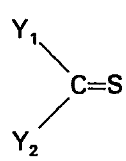

(V)

dans lesquelles:

$R_4$ est un atome d'hydrogène ou un groupe de protection éliminable,

$Y_1$ et $Y_2$ sont identiques ou différents et sont des restes réactifs, et on élimine ensuite un groupe de protection $R_4$ éventuellement présent,

ou encore,

c) un composé de formule générale VI

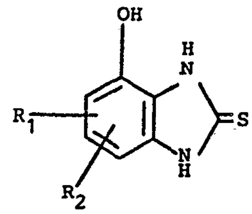

(VI)

avec un composé de formule générale VII

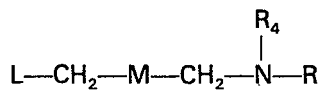

(VII)

dans lesquelles:

M est le groupe

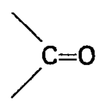

ou

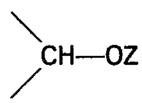

où Z a la signification du substituant $R_3$ ou peut former ensemble avec L une simple liaison et le rest L est un reste réactif,

au cas où M est le groupe

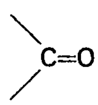

ou soumet le produit réactionnel à une réduction et on élimine un groupe de protection $R_4$ éventuellement présent,

et en ce qu'ensuite, après les réactions, on transforme éventuellement des composés de formule générale I en sels pharmacologiquement inoffensifs, en soumettant éventuellement d'abord dans les composés de formule générale I dans laquelle $R_3$ est un atome d'hydrogène, le groupe hydroxyle à une acylation.

3. Médicament contenant un composé répondant à la définition de la revendication 1, ainsi que des substances auxiliaires et de support pharmacologiquement inoffensives en soi connues.

4. Composés selon la revendication 1 ainsi que leurs sels pour l'application dans les traitements thérapeutiques ou prophylactiques des maladies due coeur et de la circulation sanguine.